# EUROPEAN PATENT APPLICATION

(11) **EP 4 477 094 A1**
(43) Date of publication of application: **18.12.2024**
(21) Application number: 24180727.0
(22) Date of filing: 07.06.2024
(51) Int. Cl.: A24D 3/04, A24D 3/17, A61M 11/00

(54) **FILTRATION ASSEMBLY AND ATOMIZER**

(30) Priority: 09.06.2023 CN 202321477685 U
(71) Applicant: Shenzhen Matrix Technical Service Co., Ltd., Shenzhen City, Guangdong Province 518101 (CN)
(72) Inventor: WANG, Ju, SHENZHEN CITY, 518101 (CN); ZENG, Fan, SHENZHEN CITY, 518101 (CN)
(74) Representative: Cabinet Beaumont

(57) **Abstract**

The present invention is about a filtration assembly and an atomizer. The filtration assembly is applied to the atomizer. The atomizer includes a housing having a gas outlet section and an atomization cavity in communication with the gas outlet section. The filtration assembly includes a filtration mesh and a flow guide rod. The filtration mesh is disposed at the gas outlet section and includes a first end away from the atomization cavity and a second end close to the atomization cavity. A shape and size of the first end match a shape and size of the gas outlet section to enable the gas outlet section to be covered. Droplets with large particle sizes in the gas outlet section is filtered by the filtration mesh and may flow from the second end of the filtration mesh to the first end of the filtration mesh, and then flow back to the atomization cavity through the flow guide rod in below to continue atomization, to prevent accumulation of droplets on the filtration mesh from causing blocking of the filtration mesh, thereby ensuring that the atomizer still has a good filtration effect after long-term use.

## Description

### FIELD

The present disclosure relates to the technical field of atomization devices, and in particularly, to a filtration assembly and an atomizer.

### BACKGROUND

An electronic atomizer generates an atomization gas in an atomization core, and the atomization gas flows to a gas outlet through a gas outlet channel. However, the atomization gas is limited by factors such as a power of the electronic atomizer and a composition of an atomization liquid. Therefore, it is difficult to limit the formation of droplets with large particle sizes during atomization. In the related art, a filtration cotton core is provided on the gas outlet channel to limit particle sizes of droplets passing through the gas outlet. However, after a long period of use, the droplets with large particle sizes are gathered on the filtration cotton core, which will cause blocking of the gas outlet channel, thereby making the electronic atomizer lose normal operation performance.

### SUMMARY

Embodiments of the present disclosure provide a filtration assembly and an atomizer to solve at least one existing technical problem described above.

An embodiment of the present disclosure provides a filtration assembly, applied to an atomizer. The atomizer includes a housing having a gas outlet section and an atomization cavity in communication with the gas outlet section. The filtration assembly includes a filtration mesh and a flow guide rod. The filtration mesh is disposed at the gas outlet section and has a first end away from the atomization cavity and a second end close to the atomization cavity. A shape and size of the first end match a shape and size of the gas outlet section to enable the gas outlet section to be covered. The size of the first end is greater than the size of the second end. The flow guide rod is connected to the second end and the atomization cavity.

In the filtration assembly described above, the filtration mesh is disposed in the gas outlet section of the atomizer, and the flow guide rod is disposed below the filtration mesh. According to flow characteristics of an atomization gas in the gas outlet section, droplets with large particle sizes in the gas outlet section filtered by the filtration mesh may flow from the second end of the filtration mesh to the first end of the filtration mesh, and then flow back to the atomization cavity through the flow guide rod in below to continue atomization, to prevent accumulation of droplets on the filtration mesh from causing blocking of the filtration mesh, thereby ensuring that the atomizer still has a good filtration effect after long-term use.

In some embodiments, the filtration mesh is in a tapered shape, the first end is a bottom surface of the tapered shape, and the second end is a tip of the tapered shape.

In some embodiments, the gas outlet section is in a cylindrical shape, and the filtration mesh is in a conical shape. A central axis of the gas outlet section coincides with a central axis of the filtration mesh.

In some embodiments, the filtration mesh is a hydrophobic filtration mesh.

In some embodiments, the filtration mesh is a mesh woven from hydrophobic microfibers. A diameter of the hydrophobic microfibers is smaller than a diameter of a mesh opening of the filtration mesh to allow for passing of atomized droplets.

In some embodiments, the mesh opening of the filtration mesh is in a polygonal shape.

In some embodiments, the flow guide rod includes a base and a body fixed at the base, the body having an insertion groove. The second end is partially accommodated in the insertion groove.

In some embodiments, the body is in a tapered shape. An end of the body close to the base is a bottom surface of the tapered shape, and an end of the body away from the base is a tip of the tapered shape.

In some embodiments, the body includes a first-level retractable rod, a second-level retractable rod, and a third-level retractable rod. The first-level retractable rod is retractably connected to the second-level retractable rod. The second-level retractable rod is retractably connected to the third-level retractable rod. The insertion groove is formed at the third-level retractable rod, and the first-level retractable rod is fixedly connected to the base.

In some embodiments, the flow guide rod has a surface formed into a hydrophilic layer.

The atomizer according to the embodiments of the present disclosure includes a housing and the filtration assembly according to any one of the above embodiments. The housing has a gas outlet section and an atomization cavity in communication with the gas outlet section. The filtration assembly is mounted at a position where the gas outlet section is in communication with the atomization cavity.

In the above atomizer provided with the filtration assembly, droplets with a particle size greater than a diameter of the filtration mesh opening can be filtered from the atomization gas. The filtered droplets with large particle sizes are drained into the atomization cavity by a flow guide tube to continue the atomization, to prevent the blocking of the filtration mesh from being blocked by the accumulation of droplets on the filtration mesh, thereby ensuring that the atomizer still has a good filtration effect after long-term use.

In some embodiments, the filtration mesh disposed at the gas outlet section has a first end away from the atomization cavity and a second end close to the atomization cavity. The gas outlet section has a recess, and a periphery of the first end has a boss engaged with the recess to limit a position of the filtration mesh in the gas outlet section.

In some embodiments, the housing has a gas outlet. The gas outlet is in communication with the atomization cavity through the gas outlet section, and a distance between the first end of the filtration mesh and the gas outlet is greater than or equal to 10 millimeters.

Additional aspects and advantages of the present disclosure will be provided in part in the following description, or will become apparent in part from the following description, or can be learned from practicing of the present disclosure.

### BRIEF DESCRIPTION OF THE DRAWINGS

In order to clearly explain technical solutions according to embodiments of the present disclosure or in the related art, drawings needing to be used in the description of the embodiments or the related art are briefly described below. Obviously, the drawings described below are merely some embodiments of the present disclosure. Based on these drawings, other drawings can be obtained by those skilled in the art without creative effort.
FIG. 1 is a schematic view showing a structure of an atomizer according to an embodiment of the present disclosure;
FIG. 2 is an enlarged view of Part II of the atomizer in FIG. 1;
FIG. 3 is a schematic view showing a structure of a flow guide rod according to an embodiment of the present disclosure; and
FIG. 4 is a schematic view showing a structure of a filtration assembly according to an embodiment of the present disclosure.

Reference Signs of components:
filtration assembly-10, filtration mesh-12, flow guide rod-14, first end-16, second end-18, base-20, body-22, insertion groove-24, first-level retractable rod-26, second-level retractable rod-28, third-level retractable rod-30, first-level spring-32, second-level spring-34, recess-36, boss-38;
atomizer-100, housing-102, gas outlet section-104, atomization cavity-106, gas outlet-108, gas inlet section-110, gas inlet-112, atomization core-114, upper cover-116.

### DETAILED DESCRIPTION

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative merely, and are intended to explain, rather than limiting, the present disclosure.

In the description of the present disclosure, it needs to be understood that, orientation or position relationship indicated by terms such as "longitudinal", "lateral", "length", "width", "thickness", "over", "below", "front", "back", "left", "right", "vertical", "horizontal", "top", "bottom", "in", "out", "clockwise", and "anti-clockwise", is based on the orientation or position relationship shown in the accompanying drawings, and is merely for the convenience of describing the present disclosure and simplifying the description, rather than indicating or implying that the associated device or element must have a specific orientation, or be constructed and operated in a specific orientation, and therefore cannot be understood as a limitation on the present disclosure. In addition, terms such as "first" and "second" are used herein for purposes of description and are not intended to indicate or imply relative importance or significance. Thus, features defined by "first" and "second" may explicitly or implicitly include one or more of such features. In the description of the present disclosure, the term "plurality" means two or more, unless defined otherwise explicitly and specifically.

In the description of the present disclosure, it needs to be noted that, unless otherwise clearly specified and limited, the terms "installed", "coupled", and "connected" should be understood in a broad sense. For example, it may be a fixed connection or a detachable connection, or a connection as one piece; a mechanical connection or an electrical connection; a direct connection or an indirect connection through an intermediate; internal communication of two components or an interaction relationship between two components. For those skilled in the art, the specific meaning of the above-mentioned terms in the embodiments of the present disclosure can be understood according to specific circumstances.

In the present disclosure, unless expressly stipulated and defined otherwise, the first feature "on" or "under" the second feature may include that the first feature is in direct contact with the second feature, or further include that the first and second features are in indirect contact through another feature between the first and second features. Moreover, the first feature "above" the second feature may mean that the first feature is directly above or obliquely above the second feature, or simply mean that the level of the first feature is higher than that of the second feature. The first feature "below" the second feature may mean that the first feature is directly below or obliquely below the second feature, or simply mean that the level of the first feature is smaller than that of the second feature.

Various embodiments or examples for implementing different structures of the present disclosure are provided below. In order to simplify the description of the present disclosure, components and configurations of specific examples are described below. These specific examples are merely for the purpose of illustration, rather than limiting the present disclosure. Further, the same reference numerals and/or reference letters may appear in different examples of the present disclosure for the purpose of simplicity and clarity, instead of indicating a relationship between different embodiments and/or the discussed configurations. In addition, the present disclosure provides examples of various specific processes and materials. However, applications of other processes and/or the use of other materials are conceivable for those of ordinary skill in the art.

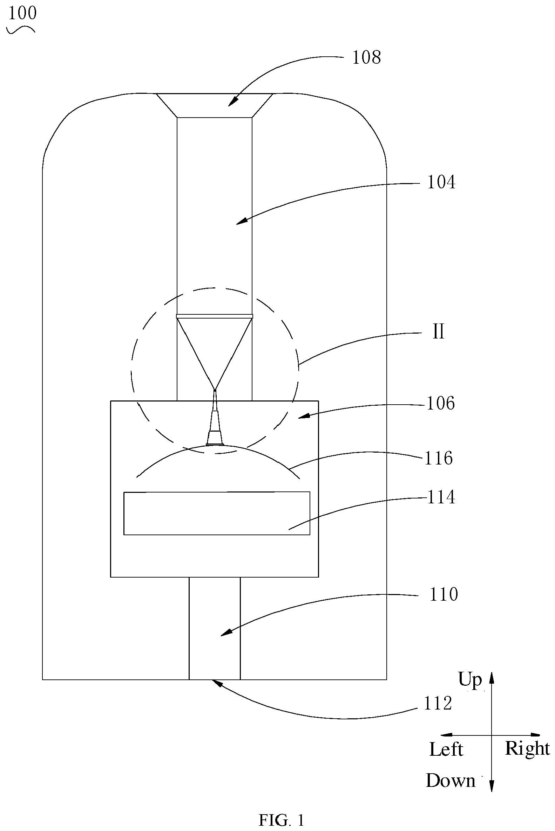

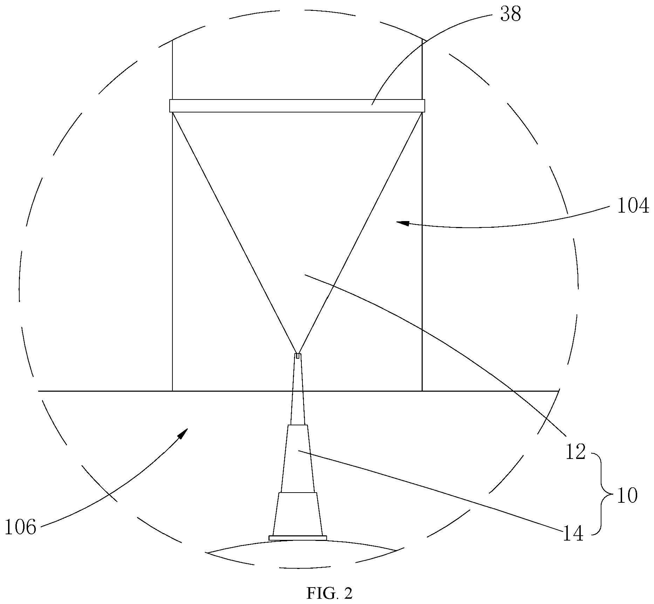

Referring to FIG. 1 and FIG. 2, an embodiment of the present disclosure provides a filtration assembly 10 applied to an atomizer 100. The atomizer 100 includes a housing 102. The housing 102 has a gas outlet section 104 and an atomization cavity 106 in communication with the gas outlet section 104. The filtration assembly 10 includes a filtration mesh 12 and a flow guide rod 14.

The filtration mesh 12 is disposed at the gas outlet section 104 and has a first end 16 away from the atomization cavity 106 and a second end 18 close to the atomization cavity 106. A shape and size of the first end 16 match a shape and size of the gas outlet section 104 to enable the gas outlet section 104 to be covered. The size of the first end 16 is greater than the size of the second end 18. The flow guide rod 14 is connected to the second end 18 and the atomization cavity 106.

In the filtration assembly 10 described above, the filtration mesh 12 is disposed in the gas outlet section 104 of the atomizer 100, and the flow guide rod 14 is disposed below the filtration mesh 12. According to flow characteristics of an atomization gas in the gas outlet section 104, droplets with large particle sizes in the gas outlet section 104 filtered by the filtration mesh 12 may flow from the second end 18 of the filtration mesh 12 to the first end 16 of the filtration mesh 12, and then flow back to the atomization cavity 106 through the flow guide rod 14 in below to continue atomization, to prevent accumulation of droplets on the filtration mesh 12 from causing blocking of the filtration mesh 12, thereby ensuring that the atomizer 100 still has a good filtration effect after long-term use.

Specifically, after the atomization gas is formed in the atomization cavity 106, the atomization gas flows to the gas outlet section 104. In order to prevent droplets with large particles from directly flowing out of the gas outlet section 104 due to insufficient atomization of the atomization gas, the filtration mesh 12 is provided at a connection of the atomization cavity 106 and the gas outlet section 104 to separate droplets with a particle size greater than a mesh opening in the atomization gas.

The atomization gas exhibits Poiseuille flow in the gas outlet section 104, i.e., the atomization gas has a high flow rate at a position close to an axis of the gas outlet section 104 and a low flow rate at a position away from the axis of the gas outlet section 104. The flow rate of the atomization gas is minimum at a position close to an inner wall surface of the housing 102 having the gas outlet section 104 formed thereon.

Therefore, the filtration assembly 10 capable of filtering the droplets with large particles from the atomization gas is designed for the gas outlet section 104 of the atomizer 100. According to the flow characteristics of the atomization gas in the gas outlet section 104, the filtration mesh 12 is provided in the gas outlet section 104 and closes the gas outlet section 104 in a direction close to the gas outlet 108 to ensure that the atomization gas passing through the gas outlet section 104 can be filtered by the filtration mesh 12. In a direction close to the atomization cavity 106, a size of the filtration mesh 12 gradually decreases, and the atomization gas flows from the atomization cavity 106 to the gas outlet 108. When passing through the filtration mesh 12, the droplets with large particle sizes filtered by the filtration mesh 12 flow towards the atomization cavity 106 along the gradually decreasing filtration mesh 12 due to the flow characteristics. The flow guide rod 14 is provided below the filtration mesh 12. The droplets flowing along the filtration mesh 12 return to the atomization cavity 106 through a flow guide effect of the flow guide rod 14, and the atomization continues. In this way, the filtration assembly 10 is provided according to the flow characteristics of the atomization gas, which can prevent the filtered-out droplets with large particle sizes from accumulating on the filtration mesh 12, thereby preventing the filtration mesh 12 from being blocked, and thus preventing the atomizer 100 from having poor gas output.

The atomization gas with a velocity gradient, when flowing through the filtration mesh 12, filters out the droplets with large particle sizes through the filtration mesh 12, and the droplets with large particle sizes has a gradient evaporation phenomenon on a surface of the filtration mesh 12, i.e., evaporation of the droplets at a side towards the axis of the gas outlet section 104 is strong, and evaporation of the droplets at a side towards the inner wall surface of the housing 102 is weak. In an embodiment, main components of an atomization liquid are propylene glycol (PG) and glycerin (VG). The PG has a surface tension smaller than a surface tension of the VG and is more likely to evaporate. Therefore, a surface tension at the side of the droplets facing towards the axis of the gas outlet section 104 is locally increased because of more decrease in PG. Affected by the Marangoni effect, all droplets are drawn up towards the axis of the gas outlet section 104 by a greater surface tension. The Marangoni effect is a phenomenon that the liquid flows from an interface with a low surface tension to an interface with a high surface tension due to the occurrence of a tension gradient between interfaces of two liquids with different surface tensions.

Referring to FIG. 2, in some embodiments, the filtration mesh 12 is in a tapered shape, the first end 16 is a bottom surface of the tapered shape, and the second end 18 is a tip of the tapered shape.

In this way, the tapered filtration mesh 12 has a good flow guide effect, which can effectively prevent the filtration mesh 12 from being blocked by the accumulation of droplets on the filtration mesh 12.

Specifically, the tapered shape may include a pyramid or a cone. The filtration mesh 12 is arranged to be a tapered shape, so that the flow characteristics of the atomization gas in the gas outlet section 104 can be better utilized. The atomization gas has a large flow rate in a direction close to the tip of the tapered shape and a small flow rate in a direction close to the bottom surface of the tapered shape, which can enable the droplets with large particle sizes filtered by the filtration mesh 12 to flow from a bottom surface of the tapered filtration mesh 12 to a tapered center. In this way, the filtration mesh 12 can be effectively prevented from being blocked because of the accumulation of droplets on the filtration mesh 12. Meanwhile, the filtration mesh 12 can be kept clean, to prolong a service life of the filtration mesh 12.

In some embodiments, the gas outlet section 104 is in a cylindrical shape, and the filtration mesh 12 is in a conical shape. A central axis of the gas outlet section 104 coincides with an axis of the filtration mesh 12.

In this way, processing and engagement of the filtration mesh 12 and the gas outlet section 104 are made simpler.

Specifically, the central axis of the gas outlet section 104 coincides with the axis of the filtration mesh 12. The central axis of the gas outlet section 104 coincides with the axis of the filtration mesh 12 completely, or a deviation between the central axis of the gas outlet section 104 and the axis of the filtration mesh 12 is within a predetermined range. In this way, an internal gas flow of the gas outlet section 104 is allowed to form a stable pipe Poiseuille flow, enabling a flow speed of an atomization gas at an axis position of the gas outlet section 104 to be maximum. Meanwhile, a cross section of the gas outlet section 104 is circular, so that a flow area of the gas outlet section 104 is larger under a same volume of the atomizer 100, and a flow effect of the atomization gas in the gas outlet section 104 is better simultaneously.

In some embodiments, the filtration mesh 12 is a hydrophobic filtration mesh.

In this way, the filtered droplets on the filtration mesh 12 do not easily block the filtration mesh 12, and the filtration mesh 12 made of a hydrophobic material has a good flow guide effect.

Specifically, the filtration mesh 12 is mainly used for filtering droplets with different particle sizes from the atomization gas, making droplets with particle sizes greater than a predetermined range be blocked, to ensure that the droplets in the atomization gas passing through the gas outlet section 104 have more uniform particle size.

When the droplet stably exists on a solid surface, the droplet will present different morphologies, which are expressed by a contact angle (CA). The CA refers to a tangent line of a gas-liquid interface made at an intersection point of gas, liquid, and solid. An angle between the tangent line on a liquid side and a solid-liquid interface line ranges from 0° to 180°. A critical CA of hydrophilicity and hydrophobicity is 90°. Being greater than 90° is defined as hydrophobic. The larger the CA, the better hydrophobic performance of the surface. A material of the filtration mesh 12 is selected as a hydrophobic material having a CA greater than 90°, including a super-hydrophobic material having a CA greater than 150°. The filtration mesh 12 may use a polymer coating, i.e., the hydrophobic performance of the material is enhanced by coating the polymer coating on a surface of a substrate, and the polymer coating includes polyethylene, polytetrafluoroethylene, and the like. Alternatively, the filtration mesh 12 may be made of a nano-material, and a material prepared by nanotechnology has a unique surface structure and physical properties, which can effectively inhibit contact of water molecules.

The filtration mesh 12 is in direct contact with the atomization gas in the gas outlet section 104, and uses the hydrophobic material, which is beneficial to keep the filtration mesh 12 clean, while preventing the filtration mesh 12 from being blocked by the droplets because of the accumulation of droplets on the filtration mesh 12.

In some embodiments, the filtration mesh 12 is a mesh woven from hydrophobic microfibers. A diameter of the hydrophobic microfibers is smaller than a diameter of a mesh opening of the filtration mesh 12 to allow for passing of atomized droplets.

In this way, a flow resistance of the atomization gas in the gas outlet section 104 is reduced.

Specifically, all the droplets with large particle sizes trapped by the filtration mesh 12 are subjected to a gradient evaporation effect caused by the Poiseuille flow, and the Marangoni effect arising from this causes liquid droplets to converge towards a tapered tip of a flue filtration mesh 12, preventing the droplets from adsorbing on the surface of the filtration mesh 12 and causing the atomization gas to block and fail to pass through the filtration mesh 12. The diameter of the hydrophobic microfibers is smaller than sizes of droplets allowed to pass through the mesh opening of the filtration mesh 12, which is beneficial to ensure a sufficient flow area, suppress an increase in a pressure drop caused by the filtration mesh 12 on the flow of the atomization gas, and ensure that a suction resistance change is little during suction of the atomization gas at the gas outlet 108.

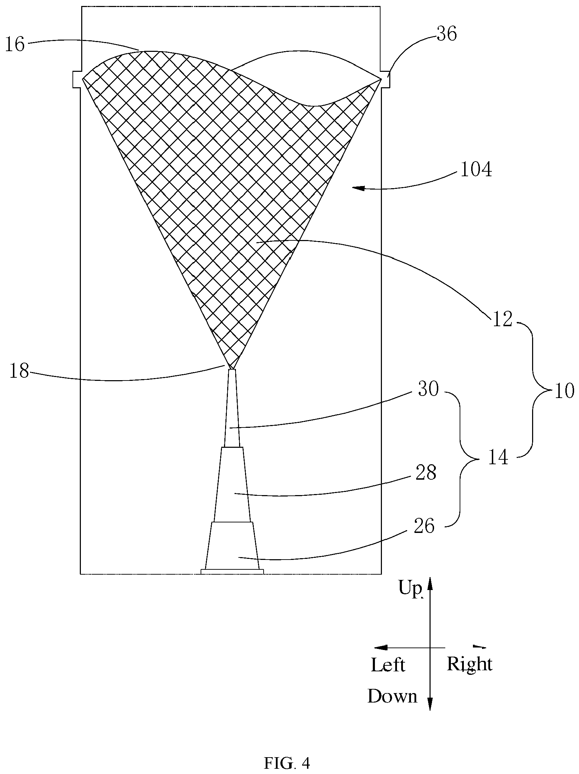

Referring to FIG. 4, in some embodiments, the mesh opening of the filtration mesh 12 is in a polygonal shape.

In this way, it is beneficial to a reduction in an on-way resistance during convergence of the droplets on the surface of the filtration mesh 12 towards the tip.

Specifically, mesh opening cells on the surface of the filtration mesh 12 are in a shape of polygons such as rhombus, honeycomb, hexagon, and triangle with angles in a vertical direction (such as an up-down direction in FIG. 4) smaller than angles in a horizontal direction (such as a left-right direction in FIG. 4), which is beneficial to a reduction in an on-way resistance during convergence of droplets on a surface of the flue filtration mesh 12 to the tip. In an embodiment, an equivalent diameter of the mesh opening cell on the surface of the filtration mesh 12 is smaller than or equal to 5 um. In an embodiment, the equivalent diameter of the mesh opening cell on the surface of the filtration mesh 12 is smaller than or equal to 1 um. The states of the atomization gas at filtration positions of an equivalent diameter of mesh opening cells on surfaces of different filtration meshes 12 have different states. The equivalent diameter of the mesh opening cell may be determined according to actual needs, which is not specifically limited herein.

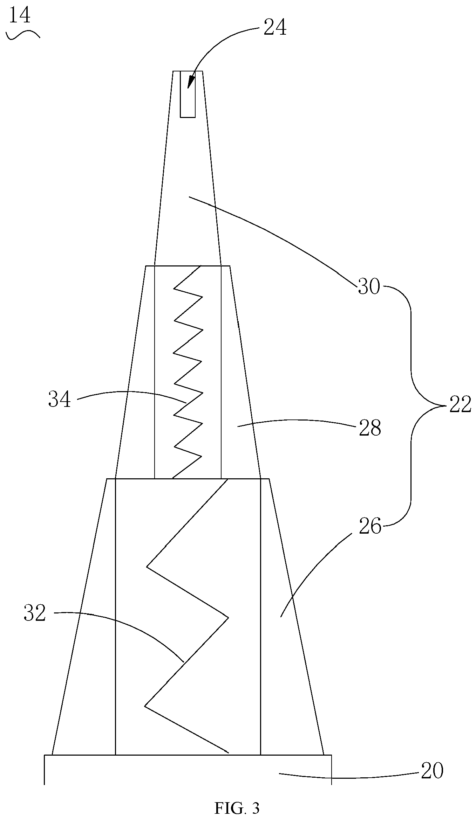

Referring to FIG. 3, in some embodiments, the flow guide rod 14 includes a base 20 and a body 22. The body 22 is fixed at the base 20 and has an insertion groove 24. A tip part of the filtration mesh 12 is accommodated in the insertion groove 24.

In this way, the filtration mesh 12 and the flow guide rod 14 are fixed by the insertion groove 24 to facilitate installation. Meanwhile, a structure of the filtration assembly 10 is made more stable.

Specifically, the flow guide rod 14 is disposed in the atomization cavity 106 for guiding flow of the filtered droplets with large particle sizes into the atomization cavity 106 for re-atomization. The filtration mesh 12 is disposed at an upper end of the body 22. The insertion groove 24 is formed at the upper end of the body 22. The second end 18 of the filtration mesh 12 is inserted in the insertion groove 24 to fix the filtration mesh 12 and the flow guide rod 14, to facilitate assembly of the filtration assembly 10, while maintaining structural stability of the filtration assembly 10.

Referring to FIG. 3, in some embodiments, the body 22 is in a tapered shape. An end of the body 22 close to the base 20 is a bottom surface of the tapered shape, and an end of the body 22 away from the base 20 is a tip of the tapered shape.

In this way, the tapered flow guide rod 14 has a better flow guide effect.

Specifically, an end of the tip of the flow guide rod 14 is connected to the filtration mesh 12, and droplets flowing from the filtration mesh 12 need to flow back to the atomization cavity 106 through the flow guide rod 14. The flow guide rod 14 is arranged in a tapered shape, so that the droplets spontaneously flow to the base 20 of the flow guide rod 14 under the driving of capillary action. In this way, the flow guide effect of the flow guide rod 14 is better, and continuous accumulation of aggregated droplets in the filtration mesh 12 is prevented. The capillary action refers to a phenomenon where a combination of an adhesion force and surface tension between the liquid and an object allows the liquid to flow to a thin tubular obj ect without the need for exertion of an external force.

Referring to FIG. 3, in some embodiments, the body 22 includes a first-level retractable rod 26, a second-level retractable rod 28, and a third-level retractable rod 30. The first-level retractable rod 26 is retractably connected to the second-level retractable rod 28. The second-level retractable rod 28 is retractably connected to the third-level retractable rod 30. The insertion groove 24 is formed at the third-level retractable rod 30, and the first-level retractable rod 26 is fixedly connected to the base 20.

In this way, the flow guide rod 14 is retractable, so that the flow guide rod 14 can be installed in atomizers 100 of different types, with wider applicability.

Specifically, in an embodiment, the first-level retractable rod 26 and the second-level retractable rod 28 have through holes formed therein. The second-level retractable rod 28 and the third-level retractable rod 30 successively pass through the through holes. The flow guide rod 14 further includes a first-level spring 32 and a second-level spring 34. The first-level spring 32 is disposed in the through hole of the first-level retractable rod 26 and abuts against a bottom of the second-level retractable rod 28, so that the second-level retractable rod 28 is retractable relative to the first-level retractable rod 26. The second-level spring 34 is disposed in the through hole of the second-level retractable rod 28 and abuts against a bottom of the third-level retractable rod 30, so that the third-level retractable rod 30 is retractable relative to the second-level retractable rod 28. In this way, the flow guide rod 14 can be installed in the atomizers 100 of different types, and has wider applicability. The first-level spring 32 and the second-level spring 34 may select springs with lower rigidity, to reduce a reaction force of the flow guide rod 14 on the filtration mesh 12, which makes the flow guide rod 14 more easily retractable to adapt to atomizers 100 of different types and sizes.

In some embodiments, the flow guide rod 14 has a surface formed into a hydrophilic layer.

In this way, the flow guide effect of the flow guide rod 14 is made better.

Specifically, the flow guide rod 14 is mainly used for draining the droplets with large particle sizes filtered by the filtration mesh 12 into the atomization cavity 106 to continue atomization. A surface of the flow guide rod 14 is selected to be made of a hydrophilic material with a CA smaller than 90°, and the hydrophilic material may be selected from materials such as metal or glass. It may also be that a hydrophilic coating, such as a polyacrylic acid coating and a silica hydrophilic coating, is used on the substrate of the flow guide rod 14.

The use of the hydrophilic material is beneficial for flow guide of an inflow, by the flow guide rod 14 through capillary action, of an e-liquid gathered at the tip of the filtration mesh 12 to the base 20 of the flow guide rod 14, so that the flow guide effect of the flow guide rod 14 is better.

In summary, the filtration assembly 10 is disposed in the gas outlet section 104 of the atomizer 100, the filtration mesh 12 is used for filtering the droplets with large particle sizes, and the flow guide rod 14 is used for being connected to an end of the tip of the filtration mesh 12, to drain the droplets gathered at the tip of the filtration mesh 12 back into the atomization cavity 106 to continue atomization.

The filtration mesh 12 is a mesh woven from hydrophobic microfibers, enabling the filtration mesh 12 to remain clean and effectively filter large droplets with a particle size greater than a given particle size. A tapered design of the filtration mesh 12 further increases a contact area between the filtration mesh 12 and the atomization gas. Compared with the prior art, filtration efficiency is greatly improved, and a suction resistance during the suction of the atomization gas is significantly lowered.

Meanwhile, all the droplets with large particle sizes trapped on the surface of the filtration mesh 12 are subjected to the gradient evaporation effect caused by the Poiseuille flow, and the Marangoni effect arising from this causes the droplets to converge to the tip of the filtration mesh 12. Compared with the prior art, a problem of gas flow blockage caused by droplets being adsorbed on the surface of the filtration mesh 12 is effectively solved.

Referring to FIG. 1, provided is an atomizer 100 according to the embodiments of the present invention, including a housing 102 and the filtration assembly 10 according to any one of the above embodiments. The housing 102 has a gas outlet section 104 and an atomization cavity 106 in communication with the gas outlet section 104. The filtration assembly 10 is mounted at a position where the gas outlet section 104 is in communication with the atomization cavity 106.

In the above atomizer 100 provided with the filtration assembly 10, droplets with a particle size greater than a diameter of the opening of the filtration mesh 12 can be filtered from the atomization gas. The filtered droplets with large particle sizes are drained into the atomization cavity 106 by a flow guide tube to continue the atomization, to prevent the filtration mesh 12 from being blocked due to the accumulation of droplets on the filtration mesh 12, thereby ensuring that the atomizer 100 still has a good filtration effect after long-term use.

Specifically, the atomizer 100 includes a housing 102. The housing 102 has an atomization cavity 106 formed therein. An atomizing core 114 is provided in the atomization cavity 106 for atomizing the atomization liquid into the atomization gas. A gas inlet section 110 and a gas outlet section 104 are also formed inside the housing 102. The gas inlet section 110 has a gas inlet 112 formed thereon, and the atomization cavity 106 is in communication with the gas inlet section 110 and the gas outlet section 104. An air enters the atomization cavity 106 from the gas inlet 112 through the gas inlet section 110. After the air is mixed with the atomization gas, the air flows out through the gas outlet section 104 and the gas outlet 108 to complete an atomization process of the atomization liquid.

The filtration assembly 10 is mounted within the gas outlet section 104. In the embodiments as shown in the drawings, the atomizer 100 further includes an upper cover 116 and a base (not shown). The upper cover 116 is mounted on the base. The atomization cavity 106 is formed between the upper cover 116 and the base. The upper cover 116 has a liquid inlet hole. The flow guide rod 14 is mounted at the upper cover 116, and the droplets flowing through the flow guide rod 14 may flow directly back into the atomization cavity 106 through the liquid inlet hole.

Referring to FIG. 1, in some embodiments, the filtration mesh 12 is disposed at the gas outlet section 104 and has a first end 16 away from the atomization cavity 106 and a second end 18 close to the atomization cavity 106. The gas outlet section 104 has a recess 36, and a periphery of the first end 16 has a boss 38. The boss 38 is engaged with the recess 36 to limit a position of the filtration mesh 12 in the gas outlet section 104.

In this way, when the filtration mesh 12 is mounted to the gas outlet section 104, positioning and assembly of the filtration mesh 12 is facilitated through the engagement of the recess 36 and the boss 38.

Specifically, the first end 16 of the filtration mesh 12 is disposed within the gas outlet section 104 and substantially covers the gas outlet section 104, so that the atomization gas passing through the gas outlet section 104 can be filtered. The recess 36 is formed on the gas outlet section 104, and the periphery of the first end 16 of the filtration mesh 12 has a boss 38. When the filtration mesh 12 is installed in the gas outlet section 104, the boss 38 is engaged with the recess 36 to fix the filtration mesh 12 in the gas outlet section 104. In this way, the installation of the filtration mesh 12 is more convenient and standardized. Meanwhile, the filtration mesh 12 can be fixed in the gas outlet section 104, so that a structure of the atomizer 100 is stable.

Referring to FIG. 1, in some embodiments, the housing 102 has a gas outlet 108. The gas outlet 108 is in communication with the atomization cavity 106 through the gas outlet section 104, and a distance between the first end 16 of the filtration mesh 12 and the gas outlet 108 is greater than or equal to 10 millimeters.

In this way, the droplets are promoted to gather towards the tip.

Specifically, the farther a distance of the filtration mesh 12 from the gas outlet 108 of the gas outlet section 104, the higher a gas flow temperature, the greater a gas viscosity, the greater a gradient of a gas flow rate, the stronger the Marangoni effect, and the better a convergence effect of the droplets towards the axis of the gas outlet section 104. In this way, the surface of the filtration mesh 12 is allowed to remain self-clean all the time, preventing the droplets from blocking the gas outlet section 104. The distance between the first end 16 of the filtration mesh 12 and the gas outlet 108 may be 10 millimeters, 11 millimeters, 12 millimeters, 15 millimeters, or 20 millimeters, or any value greater than 10 millimeters. Specific values of a distance between the filtration mesh 12 and the gas outlet 108 may be designed according to a size of the atomizer 100.

In the description of this specification, descriptions with reference to the terms "an embodiment", "some embodiments", "illustrative embodiments", "examples", "specific examples", or "some examples" etc., mean that specific features, structure, materials or characteristics described in conjunction with the embodiment or example are included in at least one embodiment or example of the present disclosure. In this specification, the schematic representations of the above terms do not necessarily refer to the same embodiment or example. Moreover, the described specific features, structures, materials or characteristics may be combined in any one or more embodiments or examples in a suitable manner.

Although embodiments of the present disclosure have been illustrated and described, it is conceivable for those of ordinary skill in the art that various changes, modifications, replacements, and variations can be made to these embodiments without departing from the principles and spirit of the present disclosure. The scope of the present disclosure shall be defined by the claims as appended and their equivalent.

## Claims

1. A filtration assembly, applied in an atomizer, the atomizer comprising a housing having a gas outlet section and an atomization cavity in communication with the gas outlet section, the filtration assembly comprising:
a filtration mesh disposed at the gas outlet section, the filtration mesh having a first end away from the atomization cavity and a second end close to the atomization cavity, a shape and size of the first end matching a shape and size of the gas outlet section to enable the gas outlet section to be covered, the size of the first end being greater than a size of the second end; and
a flow guide rod connected to the second end and the atomization cavity.

2. The filtration assembly according to claim 1, wherein the filtration mesh is in a tapered shape, the first end being a bottom surface of the tapered shape, and the second end being a tip of the tapered shape.

3. The filtration assembly according to claim 1 or 2, wherein:
the gas outlet section is in a cylindrical shape; and
the filtration mesh is in a conical shape,
wherein a central axis of the gas outlet section coincides with a central axis of the filtration mesh.

4. The filtration assembly according to any of claims 1 to 3, wherein the filtration mesh is a hydrophobic filtration mesh.

5. The filtration assembly according to any of claims 1 to 4, wherein the filtration mesh is a mesh woven from hydrophobic microfibers, a diameter of the hydrophobic microfibers being smaller than a diameter of a mesh opening of the filtration mesh to allow for passing of atomized droplets.

6. The filtration assembly according to claim 5, wherein the mesh opening of the filtration mesh is in a polygonal shape.

7. The filtration assembly according to any of claims 1 to 6, wherein the flow guide rod comprises a base and a body fixed at the base, the body having an insertion groove, the second end being partially accommodated in the insertion groove.

8. The filtration assembly according to claim 7, wherein the body is in a tapered shape, an end of the body close to the base being a bottom surface of the tapered shape, and an end of the body away from the base being a tip of the tapered shape.

9. The filtration assembly according to claim 7 or 8, wherein the body comprises a first-level retractable rod, a second-level retractable rod, and a third-level retractable rod, the first-level retractable rod being retractably connected to the second-level retractable rod, the second-level retractable rod being retractably connected to the third-level retractable rod, the insertion groove being formed at the third-level retractable rod, and the first-level retractable rod being fixedly connected to the base.

10. The filtration assembly according to any of claims 1 to 9, wherein the flow guide rod has a surface formed into a hydrophilic layer.

11. An atomizer, comprising:
a housing having a gas outlet section and an atomization cavity in communication with the gas outlet section; and
the filtration assembly according to any one of claims 1 to 10, the filtration assembly being mounted at a position where the gas outlet section is in communication with the atomization cavity.

12. The atomizer according to claim 11, wherein the filtration mesh disposed at the gas outlet section has the first end away from the atomization cavity and the second end close to the atomization cavity, the gas outlet section having a recess, and a periphery of the first end having a boss engaged with the recess to limit a position of the filtration mesh in the gas outlet section.

13. The atomizer according to claim 11 or 12, wherein the housing has a gas outlet, the gas outlet being in communication with the atomization cavity through the gas outlet section, and a distance between the first end of the filtration mesh and the gas outlet being greater than or equal to 10 millimeters.
